Europäisches Patentamt

(19) European Patent Office

Office européen des brevets

(11) Veröffentlichungsnummer: **0 073 053**
**B1**

(12) **EUROPÄISCHE PATENTSCHRIFT**

(45) Veröffentlichungstag der Patentschrift: 24.10.84

(51) Int. Cl.³: **C 07 C 149/243, C 07 B 19/00**

(21) Anmeldenummer: **82107698.1**

(22) Anmeldetag: **23.08.82**

(54) Verfahren zur Trennung des Racemats S-(Carboxymethyl)-(RS)-cystein (B).

(30) Priorität: **28.08.81 DE 3134106**

(43) Veröffentlichungstag der Anmeldung:
**02.03.83 Patentblatt 83/9**

(45) Bekanntmachung des Hinweises auf die Patenterteilung:
**24.10.84 Patentblatt 84/43**

(84) Benannte Vertragsstaaten:
**AT BE CH DE FR GB IT LI NL SE**

(56) Entgegenhaltungen:
**AT - B - 320 607**
**DE - A - 2 653 332**
**DE - B - 2 045 998**
**DE - B - 2 362 687**

**THE JOURNAL OF ORGANIC CHEMISTRY, vol. 16, no. 5, 1951, MARVIN D. ARMSTRONG et al., "Thioether derivatives of cysteine and homocysteine", Seiten 749-753**
**METHODEN DER ORGANISCHEN CHEMIE, Houben-Weyl, 4. Auflage, Band IV, Teil 2, 1955, GEORG THIEME VERLAG, Stuttgart, Seiten 519-522**

(73) Patentinhaber: **Degussa Aktiengesellschaft, Weissfrauenstrasse 9, D-6000 Frankfurt am Main 1 (DE)**

(72) Erfinder: **Bethge, Horst, Schwalbenstrasse 2a, D-6450 Hanau 1 (DE)**
Erfinder: **Kleemann, Axel, Dr., Greifenhagenstrasse 9, D-6450 Hanau 9 (DE)**
Erfinder: **Martens, Jürgen, Dr., Hochstrasse 5, D-8755 Alzenau (DE)**

## Beschreibung

Die Erfindung betrifft ein Verfahren zur Trennung des Racemats S-(Carboxymethyl)-(R,S)-cystein, insbesondere zwecks Gewinnung von S-(Carboxymethyl)-(R)-cystein. Diese Substanz wird für pharmazeutische Zwecke benötigt und dient beispielsweise als Mucolyticum.

Es ist bekannt, S-(Carboxymethyl)-(R)-cystein herzustellen, indem (R)-Cystein — auch als L-Cystein bezeichnet — mit Chloressigsäure in alkalischem Medium umgesetzt wird („J. Org. Chem.", 16 (1951), 749 bis 753).

Das hierfür als Ausgangssubstanz benötige (R)-Cystein wird im allgemeinen aus keratinhaltigem Naturstoffen gewonnen. Hierzu werden diese hydrolysiert; das freigesetzte (R,R)-Cystein wird abgetrennt und zum (R)-Cystein reduziert („Org. Synth.", vol. 5 (1925), 39 bis 41; DE-OS Nr. 2653332; „J. Am. Chem. Soc.", 52 (1930), 4500). Geeignete Naturstoffe stehen jedoch nur in begrenztem Umfang zur Verfügung.

Bei der synthetischen Herstellung des Cysteins, beispielsweise aus in 2-Stellung substituierten Thiazolinen-(3) über die entsprechenden Thiazolidin-4-carbonitrile, entsteht das Racemat (R,S)-Cystein (DE-OS Nr. 2645748). Es ist bekannt, aus diesem das (R)-Cystein zu gewinnen, indem das (R,S)-Cystein mit Dicyandiamid zur (R,S)-2-Guanidin-1,3-thiazolidin-4-carbonsäure umgesetzt wird, aus dieser mit Hilfe des Kupferkomplexsalzes der (R)-Asparaginsäure die (R)-2-Guanidin-1,3-thiazolidin-4-carbonsäure abgetrennt wird und schliesslich aus dieser das (R)-Cystein abgespalten wird (DE-AS Nr. 1795021). Dieses Verfahren zur Gewinnung des (R)-Cysteins ist so umständlich und aufwendig, dass es für eine Anwendung im technischen Massstab ungeeignet ist.

Es ist nun gefunden worden, das Racemat S-(Carboxymethyl)-(R,S)-cystein mittels der optischen Isomeren des 1-Phenyläthylamins zu trennen. Während man bei den bisherigen Verfahren zunächst (R)-Cystein, gegebenenfalls durch eine umständliche Trennung des Racemats (R,S)-Cystein, gewinnt und dann das (R)-Cystein zum S-(Carboxymethyl)-(R)-cystein umsetzt, wird nunmehr zunächst das (R,S)-Cystein zum S-(Carboxymethyl)-(R,S)-cystein umgesetzt und dann dieses Racemat getrennt. Diese Trennung ist auf einfache Weise durchführbar und ergibt die optischen Isomeren des S-(Carboxymethyl)-cysteins bei hohen Ausbeuten in guter optischer und chemischer Reinheit.

Das S-(Carboxymethyl)-(R,S)-cystein wird aus dem (R,S)-Cystein in gleicher und bekannter Weise erzeugt wie das S-(Carboxymethyl)-(R)-cystein aus dem (R)-Cystein, nämlich beispielsweise durch Umsetzung mittels Chloressigsäure in alkalischem Medium nach dem in „J. Org. Chem.", 16 (1951), 749 bis 753, angegebenen Verfahren.

Erfindungsgemäss wird das S-(Carboxymethyl)-(R)-cystein mittels (R)-1-Phenyläthylamin und das S-(Carboxymethyl)-(S)-cystein mittels (S)-1-Phenyläthylamin aus dem Racemat abgetrennt. Die sich bildenden Salze aus (R)-1-Phenyläthylamin und S-(Carboxymethyl)-(R)-cystein sowie aus (S)-1-Phenyläthylamin und S-(Carboxymethyl)-(S)-cystein sind bisher nicht beschrieben. Das Salz aus (R)-1-Phenyläthylamin und S-(Carboxymethyl)-(R)-cystein ist erheblich weniger löslich als das zu diesem diastereomere Salz aus (R)-1-Phenyläthylamin und S-(Carboxymethyl)-(S)-cystein; das Salz aus (S)-1-Phenyläthylamin und S-(Carboxymethyl)-(S)-cystein ist erheblich weniger löslich als das zu diesem diastereomere Salz aus (S)-1-Phenyläthylamin und S-(Carboxymethyl)-(R)-cystein.

Zur Durchführung des erfindungsgemässen Verfahrens wird in der bei Racemattrennungen üblichen Weise vorgegangen. Das Racemat S-(Carboxymethyl)-(R,S)-cystein wird in Gegenwart von Lösungsmitteln mit dem gewünschten optischen Isomeren des 1-Phenyläthylamins zusammengebracht, und es werden dann die gebildeten diastereomeren Salze getrennt.

Die zueinander diastereomeren Salze zeigen in zahlreichen Lösungsmitteln ausreichend grosse Löslichkeitsunterschiede. Zu diesen Lösungsmitteln gehört beispielsweise Wasser. Vorzugsweise werden als Lösungsmittel primäre oder sekundäre Alkanole mit bis zu 6 Kohlenstoffatomen oder Äther verwendet und unter diesen Lösungsmittel insbesondere solche, die mit Wasser unbegrenzt mischbar sind. In Frage kommen beispielsweise Hexan-1-ol, Butan-1-ol, Methyl-tert.-butyläther, insbesondere Methanol, Äthanol, Propan-2-ol, Dioxan und Tetrahydrofuran. Die Lösungsmittel können auch in Gemischen untereinander oder in Gemischen mit Wasser angewendet werden, jedoch werden die Mischungen zweckmässigerweise so gewählt, dass die Lösungsmittel nicht mehr als eine Phase bilden.

Das Racemat S-(Carboxymethyl)-(R,S)-cystein kann in fester Form oder als Aufschlämmung oder Lösung in dem Lösungsmittel, das betreffende optische Isomere des 1-Phenyläthylamins mit einen Lösungsmittel verdünnt oder unverdünnt eingesetzt werden. Das optische Isomere des 1-Phenyläthylamins und das Racemat S-(Carboxymethyl)-(R,S)-cystein können zwar in beliebigen Mengenverhältnissen zueinander angewendet werden, es ist im allgemeinen jedoch zweckmässig, je Mol des Racemats nicht weniger als etwa 0,5 und nicht mehr als etwa 5,0 mol des optischen Isomeren zu nehmen. Vorzugsweise werden je Mol des Racemats 0,8 bis 1,1, insbesondere 1,0 mol des optischen Isomeren angewendet. Es kann bei allen Temperaturen gearbeitet werden, bei denen die Lösungsmittel in flüssiger Form vorliegen.

Zur Trennung der diastereomeren Salze wird vorzugsweise in der bei einer fraktionierten Kristallisation üblichen Weise vorgegangen. Es wird die Mischung auf erhöhte Temperatur, vorzugsweise auf Temperaturen nahe dem Siedepunkt, gebracht, so viel Lösungsmittel angewendet, dass alle Substanzen gelöst sind, und schliesslich die Lösung zur Kristallisation abgekühlt.

Aus den anfallenden Salzen aus S-(Carboxymethyl)-(R)-cystein und (R)-1-Phenyläthylamin oder S-(Carboxymethyl)-(S)-cystein und (S)-1-Phenyläthylamin wird das betreffende S-(Carboxymethyl)cystein freigesetzt, indem die Salze mit starker Säure, vorzugsweise starker Mineralsäure, wie Chlorwasserstoffsäure, behandelt werden.

*Beispiele*

Die gewonnenen, optisch aktiven Substanzen wurden jeweils auf ihren spezifischen Drehwert $[\alpha]_D^{20}$ untersucht. Dieser wird in Grad·cm³/dm·g angegeben. Prozentangaben bedeuten Gewichtsprozente.

*A. Herstellung des S-(Carboxymethyl)-(R,S)-cysteins*

Als Ausgangssubstanz diente (R,S)-Cysteinhydrochlorid, das nach dem Verfahren gemäss der DE-OS Nr. 2645748 hergestellt war. Es wurden 140 g (1 mol) dieser Substanz mit 160 g (4 mol) Natriumhydroxid in 1000 ml Wasser gelöst. Dieser Lösung wurden zunächst 3 g Natriumhydrogensulfid und dann im Verlauf von 45 min 95 g (1 mol) Monochloressigsäure zugesetzt. Die Temperatur der Mischung wurde währenddessen auf 20° C und danach 3 h lang auf 20 bis 30° C gehalten. Das Umsetzungsgemisch wurde schliesslich durch Zugabe konzentrierter wässeriger Chlorwasserstoffsäure auf den pH-Wert 3,0 eingestellt. Hierbei schied sich das S-(Carboxymethyl)-(R,S)-cystein ab. Es wurde bei 10° C abfiltriert und so lange mit Wasser gewaschen, bis es frei von Chlorionen war. Dann wurde es unter Unterdruck bei 105° C getrocknet. Die Ausbeute betrug 173 g, entsprechend 97%, bezogen auf das eingesetzte Cysteinhydrochlorid. Der Schmelzpunkt (Zersetzungspunkt) des S-(Carboxymethyl)-(R,S)-cysteins war 188 bis 192° C.

*B. Trennung des Racemats S-(Carboxymethyl)-(R,S)-cystein*

1. 100,0 g (0,56 mol) des nach A, gewonnenen Racemats S-(Carboxymethyl)-(R,S)-cystein wurden in 1500 ml Methanol suspendiert. Der Suspension wurden 50 ml Wasser und 100 ml (0,78 mol) (S)-1-Phenyläthylamin zugesetzt. Die Mischung wurde 1 h lang unter Rückfluss auf Siedetemperatur gehalten, dann langsam auf 25° C abgekühlt und unter Absaugen filtriert. Der Rückstand wurde mit 150 ml Methanol gewaschen und bei 30° C und 25 mbar getrocknet. Die gewonnene Substanz war das Salz aus S-(Carboxymethyl)-(S)-cystein und (S)-1-Phenyläthylamin. Die Ausbeute betrag 42,5 g, entsprechend 50%, bezogen auf das in dem eingesetzten Racemat enthaltene S-(Carboxymethyl)-(S)-cystein. Der spezifische Drehwert des gewonnenen Salzes war +20,5° C (c = 1 in Wasser).

Die Elementaranalyse für $C_{13}H_{20}H_2O_4S$:

Gerechnet: C 51,81 H 6,69 N 9,58 S 10,79%

Gefunden: C 51,98 H 6,71 N 9,32 S 10,68%

36,5 g des Salzes aus S-(Carboxymethyl)-(S)-cystein und (S)-1-Phenyläthylamin wurden in 100 ml Wasser gelöst. Der Lösung wurden 300 ml Methanol zugemischt, und die Mischung wurde mit konzentrierter wässeriger Chlorwasserstoffsäure auf den pH-Wert 3,0 eingestellt. Hierbei wurde das S-(Carboxymethyl)-(S)-cystein ausgefällt. Es wurde unter Absaugen filtriert, mit 30 ml kaltem Wasser gewaschen und bei 105° C und 25 mbar getrocknet. Die Ausbeute betrug 21,9 g, entsprechend 100%, bezogen auf das eingesetzte Salz. Der Schmelzpunkt (Zersetzungspunkt) des S-(Carboxymethyl)-(S)-cysteins war 188 bis 192° C und der spezifische Drehwert +33,6° (c = 10 in wässeriger Natriumhydroxidlösung, pH-Wert 6,0).

2. Es wurde wie dem Beispiel 1 verfahren, jedoch wurden statt (S)-1-Phenyläthylamin 100 ml (R)-1-Phenyläthylamin eingesetzt. Gewonnen wurde das Salz aus S-(Carboxymethyl)-(R)-cystein und (R)-1-Phenyläthylamin. Die Ausbeute betrug 42,2 g, entsprechend 50%. Der Drehwert des Salzes war −20,4° (c = 1 in Wasser).

Die Elementaranalyse für $C_{13}H_{20}H_2O_4S$:

Gerechnet: C 51,79 H 6,58 N 9,30 S 10,60%

Gefunden: C 51,98 H 6,71 N 9,32 S 10,68%

Aus 36,5 g des Salzes aus S-(Carboxymethyl)-(R)-cystein und (R)-1-Phenyläthylamin wurden 21,9 g, entsprechend 100% Ausbeute, S-(Carboxymethyl)-(R)-cystein gewonnen. Der Schmelzpunkt (Zersetzungspunkt) war 187 bis 190° C und der Drehwert −33,6° (c = 10 in wässeriger Natriumhydroxidlösung, pH-Wert 6,0.

**Patentansprüche:**

1. Trennung des Racemats S-(Carboxymethyl)-(R,S)-cystein mittels der optischen Isomeren des 1-Phenyläthylamins.

2. Salz aus S-(Carboxymethyl)-(R)-cystein und (R)-1-Phenyläthylamin.

3. Salz aus S-(Carboxymethyl)-(S)-cystein und (S)-1-Phenyläthylamin.

**Claims**

1. The resolution of the racemate S-(carboxymethyl)-(R,S)-cysteine using the optical isomers of the 1-phenylethyl amine.

2. Salt consisting of S-(carboxymethyl)-(R)-cysteine and (R)-1-phenylethyl amine.

3. Salt consisting of S-(carboxymethyl)-(S)-cysteine and (S)-1-phenylethyl amine.

**Revendications**

1. Dédoublement du racémate S-(carboxyméthyl)-(R,S)-cystéine à l'aide des isomères optiques de la 1-phényléthylamine.

2. Sel formé à partir de la S-(carboxyméthyl)-(R)-cystéine et de la (R)-1-phényléthylamine.

3. Sel formé à partir de la S-(carboxyméthyl)-(S)-cystéine et de la (S)-1-phényléthylamine.